# EUROPÄISCHE PATENTANMELDUNG

(11) **EP 1 709 932 A1**
(43) Veröffentlichungstag der Anmeldung: **11.10.2006**
(21) Anmeldenummer: 05007385.7
(22) Anmeldetag: 05.04.2005
(51) Int. Cl.: A61C 1/18, A61B 17/16

(54) **Schnellkupplung zur Verbindung medizinischer Geräte**

(71) Anmelder: W & H Dentalwerk Bürmoos GmbH, 5111 Bürmoos (AT)
(72) Erfinder: Schatz, Norbert, A-5111 Bürmoos (AT); Schmiedlechner, Karl, A-5121 Ostermiething (AT); Gollackner, Alois P., A-5303 Thalgau (AT); Harfmann, Tobias, A-5111 Bürmoos (AT); Perwein, Wolfgang, A-5112 Lamprechtshausen (AT)

(57) **Zusammenfassung**

Die Erfindung betrifft medizinische, insbesondere dentalmedizinische, und chirurgische Handstücke (1) und Anschlussstücke (2) mit ihren jeweiligen Kupplungsvorrichtungen (17, 29) zur Schnellkupplung eines Handstücks (1) mit einem Anschlussstück (2), wobei bei der Kupplung bzw. Entkupplung zumindest eine Rastnase (41) mit begrenztem Drehwinkel in einer Nut (50) läuft und die Verbindung des Handstücks (1) mit dem Anschlussstück (2) durch eine Steck-Dreh-Bewegung erfolgt (so genannte Bajonettkupplung). Erfindungsgemäß wird vorgeschlagen, die anschlussstückseitige Kupplungsvorrichtung (29) durch eine Rastnase (41), die radial federnd auf dem Anschlussstück (2) vorgespannt ist, und die handstückseitige Kupplungsvorrichtung (17) durch eine Nut (50), die an der Innenseite (43) der Außenhülse (15) des Handstücks (1) angeordnet ist, zu bilden. An die Nut (50) schließt eine Steckausnehmung (45) an, in die die Rastnase (41) einrastet, wenn das Handstück (1) an das Anschlussstück (2) gekuppelt ist, wodurch das Handstück (1) drehfest mit dem Anschlussstück (2) verbunden ist.

Die Erfindung betrifft weiters ein Herstellungsverfahren für eine derartige Schnellkupplung, bei dem die Nut (50) der handstückseitigen Kupplungsvorrichtung (17) durch Fräsen in die Innenseite (43) der Außenhülse (15) des Handstücks (1) und die Rastnase (41) der anschlussstückseitigen Kupplungsvorrichtung (29) durch Stanzen, Erodieren oder durch Materialabtrag mittels Laserstrahlung oder Wasserstrahlschneiden erzeugt werden.

## Beschreibung

Die vorliegende Erfindung betrifft medizinische, insbesondere dentalmedizinische, und chirurgische Handstücke und Anschlussstücke mit ihren jeweiligen Kupplungsvorrichtungen zur Schnellkupplung eines Handstücks mit einem Anschlussstück, wobei bei der Kupplung bzw. Entkupplung Rastnasen mit begrenztem Drehwinkel in Schlitzen oder Nuten laufen und die Verbindung des Handstücks mit dem Anschlussstück durch eine Steck-Dreh-Bewegung erfolgt. Die Erfindung betrifft weiters ein Herstellungsverfahren für eine derartige Schnellkupplung.

Eine derartige oft als Bajonettkupplung bezeichnete Schnellkupplung ist aus der EP 484 628 A1 bekannt: Das kupplungsseitige Ende des Anschlussstücks, in diesem Fall des Versorgungsschlauchs, umfasst dabei eine Hülse mit einem oder mehreren Bajonettschlitzen, die jeweils einen ersten, parallel zur Längsachse des Anschlussstücks verlaufenden Abschnitt und einen zweiten Abschnitt, der im wesentlichen rechtwinkelig, zum ersten Abschnitt angeordneten ist, umfassen. Jeder Bajonettschlitz ist somit L- oder J-förmig aufgebaut. Das kupplungsseitige Ende des Handstücks umfasst eine oder mehrere Rastnasen, hier Stifte. Beim Kupplungsvorgang wird jeder Stift mit einem Bajonettschlitz in Kontakt gebracht und durch ein Aufeinander-zu-bewegen des Handstücks und des Anschlussstücks parallel zu ihrer Längsachse bis an das Ende des ersten Abschnitts des Bajonettschlitzes geführt. Durch eine drehende Relativbewegung der beiden medizinischen Geräte zueinander folgt der Stift anschließend dem zweiten Abschnitt des Bajonettschlitzes und rastet am Ende des zweiten Abschnitts in einer Rastausnehmung ein. Um in die Rastausnehmung zu gelangen muss der Stift vor dem Einrasten einen Federarm, der axial zur Längsachse des Anschlussstücks federnd angeordnet ist, verdrängen, wobei der Federarm nach dem Einrasten des Stifts in der Rastausnehmung wieder in seine ursprüngliche Position zurückkehrt und so ein unbeabsichtigtes Lösen der beiden Geräte verhindert.

Der Vorteil von Bajonettkupplungen liegt in ihrem einfachen, schnellen und sicheren Kupplungsvorgang, der sie für medizinische Anwendungen, bei denen eine freie Drehbarkeit der beiden medizinischen Geräte relativ zueinander nicht notwendig oder nicht erwünscht ist, besonders geeignet macht.

Nachteil dieser Bajonettkupplung ist die aufwendige Herstellung: Die die Bajonettschlitze umfassende Hülse muss in einem ersten Schritt aus einem Metallzylinder durch Zerspanen (Drehen) hergestellt werden. Um eine sichere und dauerhafte Funktion des Federarms, der in einem zweiten Bearbeitungsschritt durch Fräsen des Bajonettschlitzes aus der Hülse herausgearbeitet wird, zu gewährleisten, muss der Metallzylinder aus einem hartem, gute Federeigenschaften aufweisendem Material, bevorzugt Edelstahl, bestehen. Dies erschwert jedoch wiederum das Zerspanen des Metallzylinders. Des Weiteren sind während der Montage der Bajonettkupplung die in den Bajonettschlitzen geführten Stifte zu befestigen. Dieser Montageschritt wird von den Monteuren als unangenehm und schwierig empfunden, da die Stifte und die Bohrungen, in denen sie angeordnet sind, sehr klein sind.

Der vorliegenden Erfindung liegt daher die Aufgabe zugrunde eine in ihrer Herstellung einfachere Bajonettkupplung für ein Handstück und ein daran zu kuppelndes Anschlussstück zu schaffen.

Diese Aufgabe wird gemäß der vorliegenden Erfindung durch ein Anschlussstück mit einer anschlussstückseitigen Kupplungsvorrichtung als Teil der Bajonettkupplung mit den Merkmalen des Anspruchs 1, durch ein Handstück mit einer handstückstückseitigen Kupplungsvorrichtung als Teil der Bajonettkupplung mit den Merkmalen des Anspruchs 10 sowie durch ein Herstellungsverfahren für ein Anschlussstück bzw. ein Handstück mit der jeweiligen Kupplungsvorrichtung mit den Merkmalen des Anspruchs 16 und 18 gelöst. Besonders vorteilhafte Ausführungsformen sind in den jeweiligen Unteransprüchen angeführt.

Der erfindungsgemäße Aufbau der beiden Teile der Schnellkupplung vereinfacht ihre Herstellung merkbar: Da die Nut zur Führung der Rastnase kein federndes Bauteil (Federarm in der EP 484 628 A1) umfasst, ist es nicht notwendig die Nut aus hartem, gute Federeigenschaften aufweisendem Material herauszuarbeiten. Erfindungsgemäß wird die Nut daher in der Innenseite der Außenhülse des Handstücks angeordnet. Bevorzugt erfolgt dies während der Herstellung des Handstücks durch Fräsen.

Die am Innenumfang der Außenhülse des Handstücks angeordnete Nut ermöglicht nun eine radiale Kraftwirkung der Rastnase, um das Handstück drehfest mit dem Anschlussstück zu verbinden. Erfindungsgemäß ist daher eine radial federnde, auf dem Anschlussstück angeordnete Rastnase vorgesehen, deren axiale Abmessung (Dicke der Rastnase) so bemessen ist, dass sie in vorteilhafter Weise durch Stanzen, Erodieren oder durch Materialabtrag mittels Laserstrahlung oder Wasserstrahlschneiden aus einem Metallblech oder einer Metallplatte hergestellt werden kann. Bevorzugt ist die Rastnase Teil eines Kupplungselements, das auch mehrere, bevorzugt zwei bis vier, Rastnasen sowie einen oder mehrere Federarme und / oder Schlitze und / oder Rücksprünge und / oder Höcker und / oder ein oder mehrere Bohrungen umfassen kann. In gleicher Weise ist es erfindungsgemäß möglich, das Kupplungselement aus einem Metallblech oder einer Metallplatte durch Stanzen oder durch Materialabtrag mittels Laserstrahlung herzustellen.

In einem abschließenden Montageschritt wird die Rastnase oder das Kupplungselement am Anschlussstück befestigt. Dies erfolgt bevorzugt durch Aufpressen der Rastnase oder des Kupplungselements auf das Anschlussstück oder ein Bauteil davon (Presssitz). Zusätzlich können die Rastnase oder das Kupplungselement, nachdem sie ihre endgültige Position eingenommen haben, mit einem Fixierstift drehfest am Anschlussstück befestigt werden.

Das Metallblech oder die Metallplatte, aus der die zumindest eine Rastnase gefertigt wird, kann sowohl ein eigenständiges Bauteil sein als auch einstückig mit dem Anschlussstück ausgeführt sein, zum Beispiel als Teil des Gehäuses.

Als Anschlussstück können alle mit geraden oder gewinkelten, medizinischen, dentalmedizinischen und chirurgischen Handstücken verbindbare Geräte dienen, zum Beispiel Adapter, Versorgungsschläuche, Kupplungsstücke, insbesondere jedoch Antriebe wie Luft- oder Elektromotore.

Die Erfindung wird nachfolgend anhand bevorzugter Ausführungsbeispiele und Bezug nehmend auf die beigefügten Zeichnungen erläutert:
Figur 1 zeigt ein erfindungsgemäßes gewinkeltes Handstück und eine als Antriebsmotor ausgebildete Ausführungsform eines erfindungsgemäßen Anschlussstücks, wobei Handstück und Anschlussstück nicht miteinander gekuppelt sind.
Figur 2 zeigt den distalen Endabschnitt des als Antriebsmotor ausgebildeten Anschlussstücks gemäß Figur 1.
Figur 3 zeigt eine Ausführungsform eines Kupplungselements als Teil der anschlussstückseitigen Kupplungsvorrichtung.
Figur 4 zeigt eine Ausführungsform einer handstückseitigen Kupplungsvorrichtung.

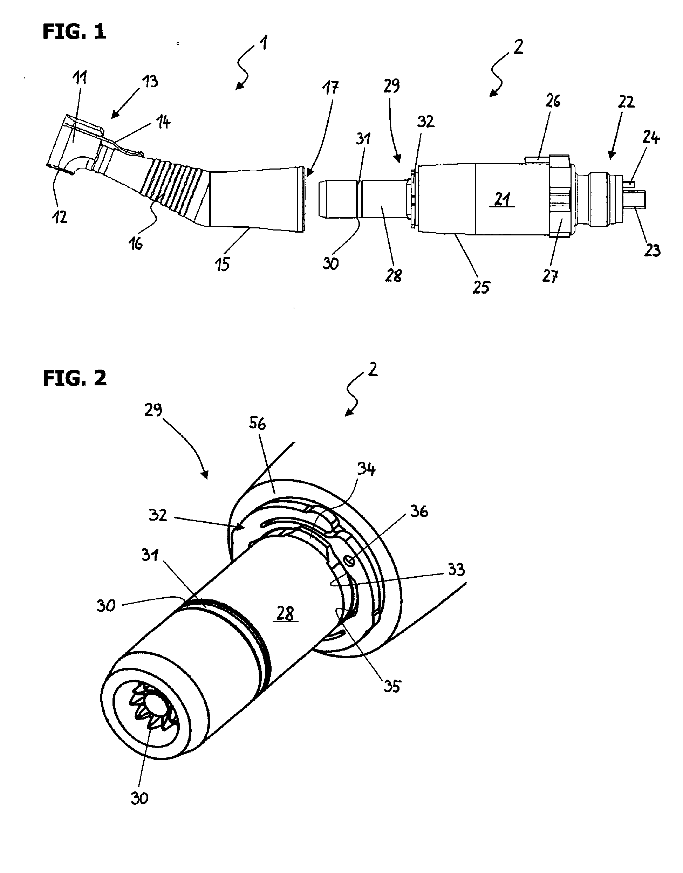

In Figur 1 sind beispielhafte Ausführungsformen des erfindungsgemäßen Handstücks 1 und des erfindungsgemäßen Anschlussstücks 2 dargestellt. Bei dem Handstück 1 handelt es sich um ein gewinkeltes Handstück zum Antrieb eines (nicht dargestellten) medizinischen Instruments, wie zum Beispiel eines rotierenden Bohrers oder einer hin- und herbewegten Feile. Selbstverständlich kann das Handstück auch als gerades Handstück ausgebildet sein. Auch kann das Handstück dem Antrieb unterschiedlichster medizinischer, dentalmedizinischer, orthopädischer und chirurgischer Instrumente dienen, zum Beispiel von Zahnsteinentfernungsinstrumenten, Bürsten, Prophy-Cups, Sägen, Shavern, Reamern etc.

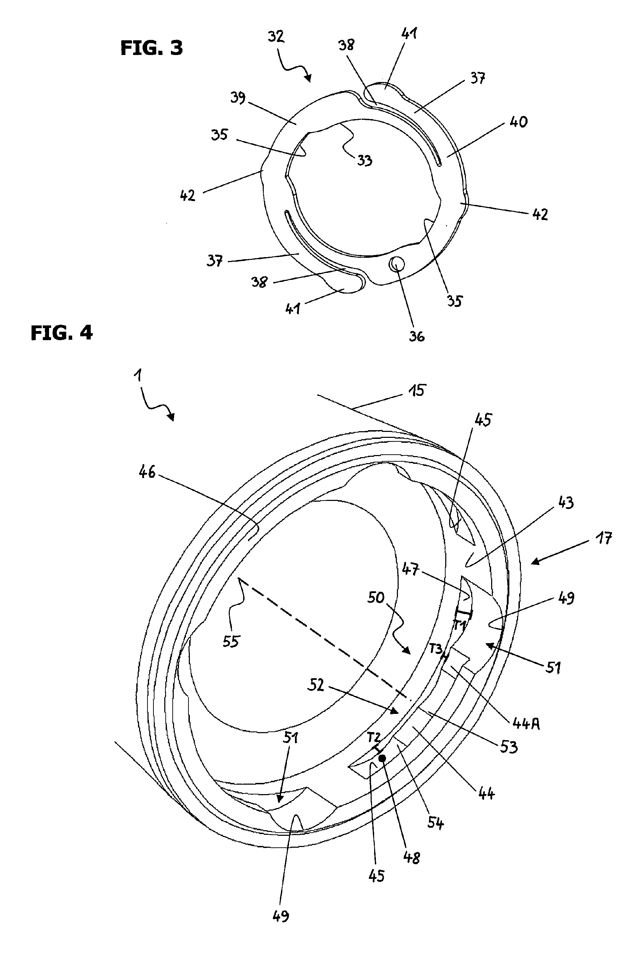

In bekannter Weise umfasst das Handstück 1 in seinem Handstückkopf 11 eine Werkzeugaufnahme, in die das anzutreibende Werkzeug durch eine am distalen Ende des Handstückkopfs 11 angeordnete Werkzeugaufnahmeöffnung 12 eingesetzt ist. Am gegenüberliegenden Ende des Handstückkopfs 11 ist eine Vorrichtung 13 zum Lösen des Werkzeugs aus der Werkzeugaufnahme angebracht, zum Beispiel ein Druckknopf oder ein drehbarer oder längs-verschiebbarer Hebel 14. Die gebogene Außenhülse 15 des Handstücks 1 hat einen Abschnitt mit einem Griffmuster 16, der dem Anwender das Halten des Handstücks 1 erleichtert. Am proximalen Ende der Griffhülse 15 ist eine handstückseitige Kupplungsvorrichtung 17, bestehend aus dem proximalen Abschnitt der Außenhülse 15 und einer Nut 50 (Figur 4) zur Verbindung des Handstücks 1 mit einem Anschlussstück 2 angeordnet.

Der Antrieb der Werkzeugaufnahme im Handstückkopf 11 kann mittels Druckluft erfolgen, die über eine Leitung im Inneren des Handstücks 1 auf einen Rotor, der mit der Werkzeugaufnahme verbunden ist, abgegeben wird. In diesem Fall ist das Anschlussstück als Versorgungsleitung ausgebildet, die mit einer Druckluftquelle verbunden ist. Alternativ kann die Werkzeugaufnahme auch mechanisch angetrieben werden, wobei eine oder mehrere, in der Außenhülse 15 angeordnete Antriebswellen, gegebenenfalls durch Getriebe miteinander verbunden, eine Antriebsbewegung, zum Beispiel eine Rotationsbewegung oder eine Vibrationsbewegung, auf die Werkzeugaufnahme übertragen.

Das in Figur 1 dargestellte Anschlussstück 2 wird durch einen Luftmotor gebildet. Es sei jedoch ausdrücklich darauf hingewiesen, dass als Anschlussstück 2 auch alle andere Geräte, die mit einem Handstück 1 verbindbar sind, verwendet werden können, zum Beispiel Adapter, Versorgungsschläuche, Kupplungsstücke und Bürsten- sowie bürstenlose Elektromotore.

Das als Luftmotor ausgebildete Anschlussstück 2 umfasst einen Mittelabschnitt 21, in dem ein mit einem Antriebsfluid, bevorzugt Druckluft, antreibbarer und dadurch in Rotation versetzbarer Rotor angeordnet ist. Am proximalen Ende des Mittelstücks ist eine Anschlussvorrichtung 22 vorgesehen, an die ein Versorgungsschlauch anschließbar ist. Über den Versorgungsschlauch wird über eine erste Leitung 23 die Antriebsdruckluft dem Rotor zugeführt und über eine zweite Leitung (nicht dargestellt) die Druckluft nach dem Passieren des Rotors wieder abgeleitet. Über zwei weitere Leitungen 24 (nur eine Leitung ist in der Abbildung zu erkennen) wird der Luftmotor mit Behandlungsfluiden, bevorzugt Wasser und Chipluft, versorgt. Diese beiden Leitungen vereinigen sich im Inneren des Anschlussstücks 2 und die darin geleiteten Medien werden zu einem Luft-Wasser-Spray vermischt, das durch eine Bohrung in der Außenhülse des Anschlussstücks 2 einer außerhalb des Anschlussstücks 2 und des Handstücks 1 verlaufenden Sprayleitung 26 zugeführt wird. Die Sprayleitung 26 ist durch einen Haltering 27 am Mittelabschnitt 21 des Anschlussstücks 2 befestigt. Alternativ kann auch nur eine Leitung 24 vorgesehen sein, die ausschließlich eine Behandlungsflüssigkeit transportiert.

Am distalen Ende des Anschlussstücks 2 befindet sich ein Kupplungszapfen 28 als Teil der anschlussstückseitigen Kupplungsvorrichtung 29, der bei Verbindung des Handstücks 1 mit dem Anschlussstück 2 im proximalen Abschnitt der Außenhülse 15 des Handstücks 1 aufgenommen wird. Am proximalen Ende des Kupplungszapfens 28 ist als weiteres Teil der anschlussstückseitigen Kupplungsvorrichtung 29 ein Kupplungselement 32 zur Verbindung des Anschlussstücks 2 mit dem Handstück 1 angeordnet. Im Inneren des hohlzylindrischen Kupplungszapfens 28 ist eine Abtriebswelle aufgenommen, die mit dem Rotor im Inneren des Anschlussstücks 2 verbunden ist und bei Druckluftzufuhr in Rotation versetzt wird. Am distalen Ende der Abtriebswelle ist ein Zahnrad 30 (Figur 2) befestigt, das, wenn das Anschlussstück 2 an das Handstück 1 gekuppelt ist, mit einem Zahnrad, das auf einer Antriebswelle im Handstück1 angeordnet ist, kämmt, wodurch die Antriebsbewegung auf die Antriebswelle und die Werkzeugaufnahme übertragen wird.

Um den Kupplungszapfen 28 verläuft ein Einstich 30, in dem ein Dämpfungselement aus elastisch-dämpfendem Material, bevorzugt ein O-Ring 31, eingesetzt ist. Ist das Anschlussstück 2 an das Handstück 1 gekuppelt, so dämpft der O-Ring 31 die Übertragung von Vibrationen vom Luftmotor auf das Handstück 1.

In Figur 2 ist das distale Ende des Anschlussstücks 2 der Figur 1 mit der anschlussstückseitigen Kupplungsvorrichtung 29 vergrößert dargestellt. Das Kupplungselement 32 weist dabei eine erste Bohrung 33 auf, deren Durchmesser so bemessen ist, dass das Kupplungselement 32 auf den Kupplungszapfen 28 geschoben und befestigt werden kann. Die Befestigung kann mittels Presssitz oder durch Klemmen erfolgen, wobei besonders bevorzugt der Kupplungszapfen 28 an seinem proximalen Ende, wo das Kupplungselement 32 fixiert ist, zumindest eine Nase 34 an seinem Außenumfang aufweist. Die erste Bohrung 33 des Kupplungselements 32 umfasst zumindest einen Rücksprung 35, dessen Form und Abmessungen der Nase 34 entsprechen. Bevorzugt sind zwei Nasen 34 und zwei Rücksprünge 35 vorhanden.

Zur Befestigung des Kupplungselements 32 am Anschlussstück 2 wird das Kupplungselement 32 über den Kupplungszapfen 28 geschoben, wobei der Rücksprung 35 auf die Nase 34 ausgerichtet wird, so dass das Kupplungselement 32 über die Nase 34 geschoben werden kann. Anschließende wird das Kupplungselement 32 relativ zum Anschlussstück 2 verdreht, wodurch die Nase 34 den Rücksprung 35 verlässt und das Kupplungselement 32 auf dem Kupplungszapfen 28 formschlüssig festklemmt. Um das Kupplungselement 32 drehfest mit dem Anschlussstück 2 zu verbinden, ist eine zweite Bohrung 36 am Kupplungselement 32 vorgesehen. Durch diese Bohrung 36 wird ein Fixierstift in eine Bohrung der Außenhülse 25 des Anschlussstücks 2 gesteckt.

In Figur 3 ist das Kupplungselement 32 aus Figur 2 vergrößert dargestellt. Es ist aus einer Metallplatte oder einem Metallblech hergestellt und hat bevorzugt eine ringförmige Form. Die erste Bohrung 33 zur Verbindung mit einem Bauteil des Anschlussstücks 2, zum Beispiel dem Kupplungszapfen 28, ist bevorzugt als zentrale, ringförmige Bohrung ausgebildet. Zwei Rücksprünge 35 dienen, wie oben beschrieben, der Befestigung des Kupplungselements 32 und der Führung während der Montage. Das Kupplungselement 32 umfasst weiters zumindest einen Federarm 37, der durch einen Schlitz 38 vom Rahmen 39 des Kupplungselements 32 beabstandet ist. Auf dem Federarm 37, bevorzugt an dem der Basis 40 des Federarms 37 gegenüberliegenden Ende, ist eine Rastnase 41 angeordnet.

Der Federarm 37 und damit die Rastnase 41 sind gegenüber dem Rahmen 39 radial, das heißt in Richtung des Rahmens 39 oder von ihm weg, beweglich. In der in der Figur 3 dargestellten unbelasteten Stellung ist die Rastnase 41 nach außen (vom Rahmen 39 wegweisend) vorgespannt. Ist das Kupplungselement 32 mit dem Anschlussstück 2 verbunden, so ist die Rastnase 41 damit in Bezug auf das Anschlussstück 2 radial nach außen federnd vorgespannt. Der Federarm 37 ist im Wesentlichen rechtwinkelig zur Längsachse des Anschlussstücks 2 angeordnet ist.

Am Außenumfang des Kupplungselements 32 ist zumindest ein Höcker 42 vorgesehen. Wie weiter unten noch detailliert beschrieben, erfolgt die Kupplung des Handstücks 1 mit dem Anschlussstück 2 durch eine Steck-Dreh-Bewegung, bei der in einem Teilschritt jede Rastnase 41 in eine Aufnahmeöffnung 49 einer Nut 50 (Figur 4) eingeschoben wird. Die Höcker 42 dienen dazu, beim Ausrichten einer Rastnase 41 auf eine Aufnahmeöffnung 49, das durch Aneinanderdrücken des Handstücks 1 und des Anschlussstücks 2 und durch Drehen der beiden Geräte relativ zueinander erfolgt, den axialen Schub, der durch das Aneinanderdrücken entsteht, aufzunehmen und dadurch die Federarme 37 zu entlasten, so dass diese nicht seitlich (bezogen auf den Rahmen 39) verbogen werden. Sind das Handstück 1 und das Anschlussstück 2 miteinander verbunden, so dienen die Höcker 42 des weiteren der axialen Stabilisierung der Behandlungsvorrichtung Handstück1 - Anschlussstück 2, da sie in die Steckausnehmung 45 (Figur 4) eingreifen und eine axiale Bewegung der Federarme 37 unterbinden.

Figur 4 zeigt vergrößert das proximale Ende des Handstücks 1 der Figur 1 mit der handstückseitigen Kupplungsvorrichtung 17. An der Innenseite 43 am Innenumfang der Außenhülse 15 weist die handstückstückseitige Kupplungsvorrichtung 17 zumindest eine Nut 50 auf. Die Nut 50 umfasst bevorzugt einen ersten Abschnitt 51 und einen zweiten Abschnitt 52, die gewinkelt zueinander angeordnet sind, wobei besonders bevorzugt beiden Abschnitte 51, 52 einen Winkel von etwa 90° einschließen. Weiters verläuft der erste Abschnitt 51 im Wesentlichen parallel zur Längsachse 55 des Handstücks 1. Alternativ kann die Nut 50 auch gebogen ausgeführt sein.

Die Nut 50, bevorzugt der erste Abschnitt 51, weist eine Aufnahmeöffnung 49 zur Aufnahme der Rastnase 41 der anschlussstückseitigen Kupplungsvorrichtung 29 auf. Weiters ist in der Nut 50, bevorzugt im zweiten Abschnitt 52, zumindest ein Rasthöcker 44, 44 A angeordnet und schließt an die Nut 50 eine Steckausnehmung 45 an. Die Nut 50, die Aufnahmeöffnung 49, der Rasthöcker 44, 44 A und die Steckausnehmung 45 werden bevorzugt durch Fräsen aus der Außenhülse 15 herausgearbeitet.

Die Verbindung des Handstücks 1 mit dem Anschlussstück 2 erfolgt in mehreren Schritten durch eine Steck-Dreh-Bewegung: Der Anwender nimmt das Handstück 1 in eine Hand und das Anschlussstück 2 in seine andere Hand. Er steckt dann die beiden Geräte mit geringem Kraftaufwand zusammen, indem er ein Bauteil des Anschlussstücks 2, im Fall des hier dargestellten Luftmotors den Kupplungszapfen 28, in das proximale Ende der Außenhülse 15 des Handstücks 1 einführt, bis die Rastnase 41 an einer proximalen Planfläche 46 der Außenhülse 15 anstößt. Anschließend dreht der Anwender das Anschlussstück 2 relativ zum Handstück 1 bis jede Rastnase 41 der anschlussstückseitigen Kupplungsvorrichtung 29 mit einer Aufnahmeöffnung 49 ausgerichtet ist und in diese und bevorzugt in den daran anschließenden ersten Abschnitt 51 der Nut 50 eingreift. Die radiale Tiefe T1 der Aufnahmeöffnung 49 und bevorzugt des gesamten ersten Abschnitts 51 der Nut 50 ist dabei so bemessen, dass die Rastnase 41 auch beim Eintritt in die Aufnahmeöffnung 49 in ihrer ursprünglichen, vom Anschlussstück 2 radial nach außen vorgespannten Stellung, wie sie in Figur 3 dargestellt ist, verbleibt.

Durch das Eingreifen der Rastnase 41 in die Aufnahmeöffnung 49 ist es dem Anwender möglich, das Handstück 1 mit dem Anschlussstück 2 weiter axial zusammen zuschieben, entweder bis die Rastnase 41 an der Begrenzungswand 47 des ersten Abschnitts 51 der Nut 50 ansteht oder bis die Planfläche 46 der Außenhülse 15 eine Kontaktfläche 56 (Figur 2) des Anschlussstücks 2 kontaktiert. Dann dreht der Anwender das Handstück 1 ein weiteres Mal relativ zum Anschlussstück 2, so dass die Rastnase 41 der Nut 50, insbesondere dem zweiten Abschnitt 52, folgt und mit begrenztem Drehwinkel in der Nut 50 des Handstücks 1 läuft, bis sie in eine Steckausnehmung 45 einrastet. Die radiale Tiefe T2 der Steckausnehmung 45 ist geringer als die Tiefe T1 der Nut 50 bzw. der Aufnahmeöffnung 49 und so bemessen, dass die vorgespannte Rastnase 41 durch die Stirnwand 48 der Steckausnehmung 45 radial nach innen (in Richtung der Längsachse 55 des Handstücks 1) gedrückt wird bzw. die Rastnase 41 nach außen gegen die Stirnwand 48 drückt, wodurch das Anschlussstück 2 drehfest mit dem Handstück 1 verbunden ist.

Ein unerwünschtes Lösen der Verbindung wird durch den Rasthöcker 44 verhindert, der zwischen der Aufnahmeöffnung 49 der Nut 50 und der Steckausnehmung 45 angeordnet ist, wobei die radiale Tiefe T3 der Nut im Bereich des Rasthöckers 44 geringer ist als die Tiefe T2 der Steckausnehmung 45. Um eine besonders sichere Verbindung zwischen dem Handstück 1 und dem Anschlussstück 2 zu gewährleisten, ist bevorzugt zwischen der Aufnahmeöffnung 49 und dem Rasthöcker 44 ein zweiter Rasthöcker 44 A vorgesehen, in dessen Bereich die Nut in etwa die selbe radiale Tiefe T3 hat wie im Bereich des ersten Rasthöckers 44. Aufgrund der geringen Tiefe T3 der Nut im Bereich der Rasthöcker 44, 44 A wird die Rastnase 41 entgegen ihrer Vorspannung besonders weit in Richtung der Längsachse 55 des Handstücks 1 gedrückt, so dass zur Überwindung der Rasthöcker 44, 44 A die während des Kupplungsvorgangs größte Kraft durch den Anwender aufzubringen ist. Dadurch erfährt der Anwender eine taktile Rückmeldung, die ihm anzeigt, dass die Rastnase 41 den / die Rasthöcker 44, 44 A überwunden hat und die Verbindung zwischen dem Handstück 1 und dem Anschlussstück 2 erfolgt ist bzw. dass sich die Rastnase 41 zwischen den beiden Rasthöckern 44 und 44 A befindet.

Das Trennen des Handstücks 1 vom Anschlussstück 2 erfolgt durch Ausführen der oben angeführten Teilschritte in umgekehrter Reihenfolge.

Um das Kuppeln des Handstücks 1 an das Anschlussstück zu erleichtern und eine ungewollte Trennung der beiden Geräte zu erschweren, können die Flanken 53, 54 eines oder beider Rasthöcker 44, 44 A unterschiedliche Neigungen aufweisen. Bevorzugt hat die näher zur Aufnahmeöffnung 49 angeordnete Flanke 53 eine geringere Neigung und die von der Aufnahmeöffnung 49 entferntere Flanke 54 eine größere Neigung.

Bevorzugt sind an der Innenseite 43 der Außenhülse 15 mehrere Nuten 50, bevorzugt zwei bis vier, besonders bevorzugt vier, angeordnet, so dass das Handstück 1 in verschiedenen Positionen im Bezug zum Anschlussstück 2 befestigt werden kann.

Die Erfindung ist nicht auf die beschriebenen Ausführungsbeispiele beschränkt, sondern umfaßt alle Ausführungsmöglichkeiten, die das prinzipielle, sinngemäße Funktionsprinzip der Erfindung nicht verändern. Insbesondere ist es im Sinn einer kinetischen Umkehr möglich, dass die Rastnase radial nach innen vorgespannt ist.

## Patentansprüche

1. Anschlussstück (2) mit einer anschlussstückseitigen Kupplungsvorrichtung (29) zur Verbindung des Anschlussstücks (2) mit einem medizinischen, insbesondere dentalmedizinischen, oder chirurgischen Handstück (1), wobei die anschlussstückseitige Kupplungsvorrichtung (29) eine Rastnase (41) umfasst, die radial federnd auf dem Anschlussstück (2) vorgespannt ist, die beim Verbinden des Anschlussstücks (2) mit dem Handstück (1) bzw. beim Lösen des Anschlussstücks (2) vom Handstück (1) mit begrenztem Drehwinkel in einer Nut (50) des Handstücks
(1) läuft und die, wenn das Anschlussstück (2) an das Handstück (1) gekuppelt ist, durch die radiale Vorspannung in eine Steckausnehmung (45) einrastet, wodurch das Anschlussstück (2) drehfest mit dem Handstück (1) verbunden ist.

2. Anschlussstück (2) nach Anspruch 1, **dadurch gekennzeichnet, dass** die Rastnase (41) auf einem Federarm (37) angeordnet ist, der im Wesentlichen rechtwinkelig zur Längsachse des Anschlussstücks (2) angeordnet ist.

3. Anschlussstück (2) nach Anspruch 2, **dadurch gekennzeichnet, dass** der Federarm (37) Teil eines Kupplungselements (32) ist und dass der Federarm (37) vom Rahmen (39) des Kupplungselement (32) durch einen Schlitz (38) beabstandet ist.

4. Anschlussstück (2) nach Anspruch 3, **dadurch gekennzeichnet, dass** das Kupplungselement (32) ringförmig ausgebildet ist und eine erste Bohrung (33) zur Verbindung mit einem Bauteil des Anschlussstücks (2) umfasst.

5. Anschlussstück (2) nach Anspruch 4, **dadurch gekennzeichnet, dass** die erste Bohrung (33) ringförmig ausgebildet ist.

6. Anschlussstück (2) nach Anspruch 4 oder 5, **dadurch gekennzeichnet, dass** die erste Bohrung (33) zumindest einen Rücksprung (35) zur Aufnahme einer Nase (34) auf jenem Bauteil des Anschlussstücks (2), das mit der Bohrung (33) verbindbar ist, aufweist.

7. Anschlussstück (2) nach einem der Ansprüche 3 bis 6, **dadurch gekennzeichnet, dass**
das Kupplungselement (32) eine zweite Bohrung (36) zur Aufnahme eines Fixierstiftes, der das Kupplungselement (32) drehfest am Anschlussstück (2) befestigt, umfasst.

8. Anschlussstück (2) nach einem der Ansprüche 3 bis 7, **dadurch gekennzeichnet, dass**
das Kupplungselement (32) zumindest einen Höcker (42) zur Aufnahme des axialen Schubs bei der Positionierung der Rastnase (41) an der Aufnahmeöffnung (49) der Nut (50) und zur axialen Stabilisierung aufweist.

9. Anschlussstück (2) nach einem der vorhergehenden Ansprüche, **gekennzeichnet durch**
mehrere, bevorzugt zwei bis vier, Rastnasen (41) und mehrere Federarme (37).

10. Medizinisches, insbesondere dentalmedizinisches, oder chirurgisches Handstück (1) mit einer handstückstückseitigen Kupplungsvorrichtung (17) zur Verbindung des Handstücks (1) mit dem Anschlussstück (2) nach einem der Ansprüche 1 - 9, wobei die handstückstückseitige Kupplungsvorrichtung (17) an der Innenseite (43) der Außenhülse (15) des Handstücks (1) eine Nut (50) zur Führung der Rastnase (41) des Anschlussstücks (2) umfasst, wobei die Nut (50) so geformt ist, dass sie beim Verbinden des Anschlussstücks (2) mit dem Handstück (1) bzw. beim Lösen des Anschlussstücks (2) vom Handstück (1) die Rastnase (41) mit begrenztem Drehwinkel führt und an die Nut (50) eine Steckausnehmung (45) anschließt, in die die radial federnde, vorgespannte Rastnase (41) einrastet, wenn das Handstück (1) an das Anschlussstück (2) gekuppelt ist, wodurch das Handstück (1) drehfest mit dem Anschlussstück (2) verbunden ist.

11. Handstück (1) nach Anspruch 10, **dadurch gekennzeichnet, dass** die Nut (50) einen ersten und zweiten Abschnitt (51, 52) umfasst, die gewinkelt zueinander angeordnet sind.

12. Handstück (1) nach Anspruch 11, **dadurch gekennzeichnet, dass** der erste und zweite Abschnitt (51, 52) einen Winkel von etwa 90° einschließen.

13. Handstück (1) nach Anspruch 11 oder 12, **dadurch gekennzeichnet, dass** der erste Abschnitt (51) im Wesentlichen parallel zur Längsachse (55) des Handstücks (1) verläuft.

14. Handstück (1) nach einem der Ansprüche 10 bis 13, **dadurch gekennzeichnet, dass** die Tiefe T2 der Steckausnehmung (45) unterschiedlich ist von der Tiefe T1 der Nut (50), bevorzugt die Tiefe T2 der Steckausnehmung (45) geringer ist als die Tiefe T1 der Nut (50), insbesondere geringer als die Tiefe der Aufnahmeöffnung (49) der Nut (50).

15. Handstück (1) nach einem der Ansprüche 10 bis 14, **dadurch gekennzeichnet, dass** die Nut (50) zumindest einen Rasthöcker (44, 44 A) umfasst, der zwischen der Aufnahmeöffnung (49) der Nut (50) und der Steckausnehmung (45) angeordnet ist, wobei die Tiefe T3 der Nut (50) im Bereich des Rasthöckers (44, 44 A) geringer ist als die Tiefe T2 der Steckausnehmung (45).

16. Verfahren zur Herstellung eines Anschlussstücks (2) mit einer anschlussstückseitigen Kupplungsvorrichtung (29), **gekennzeichnet durch** die Schritte
- zur Verfügung stellen einer Metallplatte oder eines Metallblechs,
- Herstellen zumindest einer Rastnase (41) aus der Metallplatte **durch** Stanzen, Erodieren oder **durch** Materialabtrag mittels Laserstrahlung oder Wasserstrahlschneiden.
- Befestigen der Rastnase (41) am Anschlussstück (2).

17. Verfahren zur Herstellung eines Anschlussstücks (2) nach Anspruch 16, **dadurch gekennzeichnet, dass**
des Weiteren ein Federarm (37) und / oder ein Schlitz (38) und / oder eine erste Bohrung (33) und / oder eine zweite Bohrung (36) und / oder ein Rücksprung (35) und / oder ein Höcker (42) durch Stanzen, Erodieren oder durch Materialabtrag mittels Laserstrahlung oder Wasserstrahlschneiden herausgearbeitet werden.

18. Verfahren zur Herstellung eines Handstücks (1) mit einer handstückseitigen Kupplungsvorrichtung (17), **dadurch gekennzeichnet, dass**
in die Innenseite (43) der Außenhülse (15) des Handstücks (1) eine Nut (50) zur Führung der Rastnase (41) des Anschlussstücks (2) und eine Steckausnehmung (45) gefräst werden, wobei die Nut (50) so geformt ist, dass sie beim Verbinden des Anschlussstücks (2) mit dem Handstück (1) bzw. beim Lösen des Anschlussstücks (2) vom Handstück (1) die Rastnase (41) mit begrenztem Drehwinkel führt.

19. Verfahren zur Herstellung eines Handstücks (1) nach Anspruch 18, **dadurch gekennzeichnet, dass**
die Steckausnehmung (45) so gefräst wird, dass sie eine Tiefe T2 hat, die sich von der Tiefe T1 der Nut (50) unterscheidet, wobei die Tiefe T2 der Steckausnehmung (45) bevorzugt geringer als die Tiefe T1 der Nut (50), besonders bevorzugt geringer als die Tiefe der Aufnahmeöffnung (49) der Nut (50), ist und die Nut (50) so gefräst wird, dass sie zumindest einen Rasthöcker (44, 44 A) umfasst, wobei die Tiefe T3 der Nut (50) im Bereich des Rasthöckers (44, 44 A) geringer ist als die Tiefe T2 der Steckausnehmung (45).
